# EUROPEAN PATENT APPLICATION

(11) **EP 3 517 121 A1**
(43) Date of publication of application: **31.07.2019**
(21) Application number: 17853390.7
(22) Date of filing: 19.09.2017
(51) Int. Cl.: A61K 36/899

(54) **PHARMACEUTICAL COMPOSITION COMPRISING PURPLE CORN EXTRACT FOR PREVENTION OR TREATMENT OF SKIN DISEASE**

(30) Priority: 22.09.2016 KR 20160121597; 06.03.2017 KR 20170028264
(71) Applicant: Frontbio Co. Ltd, Chuncheon-si, Gangwon-do 24232 (KR)
(72) Inventor: PARK, Soo Hyun, Chuncheon-si Gangwon-do 24267 (KR); KIM, Set Byeol, Chuncheon-si Gangwon-do 24322 (KR)
(74) Representative: Patentship Patentanwaltsgesellschaft mbH
(86) International application number: PCT/KR2017/010260
(87) International publication number: WO 2018/056676

(57) **Abstract**

Disclosed are a pharmaceutical composition for preventing or treating a skin disease containing a purple corn extract, and more particularly, to a pharmaceutical composition for preventing or treating a skin disease containing a purple corn extract that has a potent effect of preventing or treating at least one skin disease selected from the group consisting of atopy, psoriasis, eczema, keratosis, prurigo and warts, using the purple corn extract.

## Description

### [Technical Field]

The present invention relates to a pharmaceutical composition for preventing or treating a skin disease containing a purple corn extract, and more particularly, to a pharmaceutical composition for preventing or treating a skin disease containing a purple corn extract that has a potent effect of preventing or treating at least one skin disease selected from the group consisting of atopy, psoriasis, eczema, keratosis, prurigo and warts, using the purple corn extract.

### [Background Art]

Psoriasis is a chronic recurrent skin inflammatory disease that is accompanied by erythematous papules covered with silvery white scaliness. Approximately 3% (125 million) of the world's population suffer from psoriasis and about 2% of the population of Korea suffer from psoriasis. Psoriasis is one of the representative chronic inflammatory skin diseases that are difficult to treat, which is characterized by histological abnormal differentiation of the stratum corneum. Therefore, there is continuously increasing demand for psoriasis drugs.

Eczema is a term referring collectively to a group of skin diseases that have common clinical and histological characteristics. Eczema has skin symptoms such as blisters, papules, erythema and swelling, accompanied by itching, in the early stage, and has reduced swelling and blisters, but has noticeable skin wrinkles, lichenification accompanied by skin thickening, scales and pigmentation, in the chronic phase. In general, dermatitis and eczema are used as synonyms, but dermatitis is a term referring to any inflammation and in a strict definition, dermatitis has a broader meaning than eczema.

It is generally recognized that atopic dermatitis is caused by a combination of environment, food and genetic factors, although the cause of atopic dermatitis has not been clearly found yet. Atopic dermatitis is considered to be caused by a combination of a variety of factors including dry skin, skin that is itchier than normal skin, infections caused by bacteria, viruses, molds and the like, and psychological factors.

Atopic dermatitis is accompanied by pruritus as the most important symptom, and usually results in skin dryness and a vicious cycle wherein scratch to relieve pruritus leads to rashes, which causes pruritus again. The acute phase is accompanied by erythematous papules with unclear boundaries and, in serious cases, may result in edema, oozing, scratches, crusts, dryness, lichenification, infections and the like. Scratches may cause secondary infections by staphylococci, and may be accompanied by pustules and crusts. Repeated chronic skin scratches may result in lichenification, cracks accompanied by pain, and pigmentation and then darkening due to repetitive irritation. However, in addition to such symptoms, this disease may be more serious because it often causes an increased burden of medical expenses due to frequent recurrence, restriction of normal academic and social activities, depression, gangrene, suicidal impetus and the like.

In order to treat the above-mentioned skin diseases, a steroid agent is commonly used. However, it is known that steroid agents temporarily improve symptoms, but cause side effects in that the symptoms become more serious due to rebound phenomenon, when a steroid agent is discontinued after continuous use, and cause other side effects such as diabetes and hypertension.

Accordingly, Korean Patent Laid-open No. 10-2017-0013047 discloses a cosmetic composition for enhancing skin elasticity, skin moisturizing and skin whitening, and relieving atopic dermatitis containing a mixed herbal medicine extract as an active ingredient and a method for preparing the same, and Korean Patent Laid-open No. 10-2016-0146021 discloses a cosmetic composition which has excellent skin-soothing and atopic dermatitis-relieving effects, without causing any side effects due to incorporation of natural substances such as a *Centella Asiatica* extract, a *Magnolia Biondii* bark extract, a *Thujopsis Dolabrata* branch extract, an eucalyptus oil and a *Borago officinalis* seed oil.

The prior art described above can reduce side effects upon treatment of skin diseases and obtain a skin treatment effect by using a composition containing natural substance extracts. However, since a variety of natural substances should be used in combination, rather than one natural substance, the production procedure is complicated and there is a problem of increased production cost. In addition, other chemical substances, which are not intact natural substances, are used in combination, thus causing problems of secondary skin diseases.

### [Disclosure]

### [Technical Problem]

Thus, the present invention has identified that the pharmaceutical composition containing a purple corn extract is effective in the prevention or treatment of skin diseases and focused on research associated with an active ingredient having an effect of preventing or treating skin diseases. As a result, it was found that an extract of purple corn obtained using an organic solvent is potently effective for preventing or treating skin diseases.

Therefore, it is one object of the present invention to provide a pharmaceutical composition for preventing or treating a skin disease containing a purple corn extract highly effective for preventing or treating a skin disease.

### [Technical Solution]

Hereinafter, various embodiments described herein will be described with reference to the annexed drawings. In the following description, for complete understanding of the present invention, various specific details such as specific forms, compositions and processes are described. However, specific embodiments may be implemented without one or more of these specific details, or in conjunction with other well-known methods and forms. In other instances, well-known processes and manufacturing techniques are not described as certain details, in order not to unnecessarily obscure the present invention. Reference to "one embodiment" or "embodiment" throughout this specification means that a particular feature, form, composition or characteristic described in connection with the embodiment is included in at least one embodiment of the invention. Accordingly, the term "in one embodiment" or "embodiment" used in various contexts throughout this specification does not necessarily indicate the same embodiment of the present invention. In addition, a particular feature, form, composition or characteristic may be combined in any suitable manner in one or more embodiments.

Unless defined otherwise herein, all scientific and technical terms used herein have the same meaning as commonly understood by a person with ordinary skills in the art to which the present invention pertains.

In one embodiment of the present invention, provided is a pharmaceutical composition for preventing or treating skin diseases containing a purple corn extract as an active ingredient. Specifically, the purple corn may be a purple corn cob or seed, but the present invention is not limited thereto.

In the present invention, the term "purple corn (*Zea mays L*.)" refers collectively to corn, cobs or seeds of which have a dark color. The color may include not only red but also purple, brown, indigo or black. The purple corn may be variably called depending on area, classification and the influence of the dialect.

In one embodiment of the present invention, the purple corn may be a corn having any one color selected from red, purple, brown, green, indigo and black.

In one embodiment of the present invention, the skin disease may be at least one selected from the group consisting of atopy, psoriasis, eczema, keratosis, prurigo and warts.

In one embodiment of the present invention, the skin disease may be psoriasis.

In one embodiment of the present invention, the skin disease may be eczema.

In one embodiment of the present invention, the skin disease may be atopy.

As used herein, the term "extract" means an active ingredient isolated from a natural product and means herein a substance isolated from cobs or seeds of purple corn.

As used herein, the term "prevention" means any action that inhibits a skin disease or delays the development of the skin disease by administration of a composition.

As used herein, the term "treatment" means any action that relieves or favorably changes symptoms caused by a skin disease by administration of a composition.

Generally, purple corn is a perennial plant belonging to the family Gramines, which has been cultivated in Peru, Latin America, for thousands of years. In addition, purple corn contains various functional ingredients such as dietary fiber, polyphenols, plant sterols, tocopherol derivatives and carotenoids, and in particular contains anthocyanin, which is one of the polyphenols, as a main ingredient. The anthocyanin is a kind of water soluble flavonoid red pigment of polyphenol, and can be useful as a material for health functional foods and medicines owing to excellent anti-obesity, anti-diabetic and anti-oxidant effects.

The purple corn used in the present invention is a variety of corn, the entire area of which is purple in color, and is a unique variety of corn which has a small amount of grains and is purple throughout the entire area excluding the leaves.

Psoriasis is a chronic inflammatory skin disease that is accompanied by repeated formation of rashes with red papules or plaques covered with silvery white scales and having a clear boundary and a variety of sizes in the skin of the whole body, and has histological features of proliferation of epidermal keratinocytes and dermal inflammation.

Eczema refers collectively to a group of skin diseases that exhibit common clinical and histological features, which clinically have symptoms such as itching, erythema, scales and clustered papules and blisters, and histologically are superficial dermatitis, which is accompanied by spongiosis of the epidermis, and inflammatory skin reactions involved in infiltration of inflammatory cells around the blood vessels on the dermis.

Atopic dermatitis is a chronic, recurrent, inflammatory skin disease usually starting in infancy or childhood, which is accompanied by pruritus (itching), skin dryness and characteristic eczema. In the early stage, it usually induces itching, blisters, papules, erythema and edema. In the chronic phase, it has reduced swelling and blisters, but involves noticeable skin wrinkles, lichenification accompanied by skin thickening, scales and pigmentation.

In general, atopic dermatitis and eczema are synonymous, but dermatitis is a term referring to any inflammation and in a strict definition, dermatitis has a broader meaning than eczema.

Keratosis refers to a skin disease in which the stratum corneum, the upper stratum of the epidermis, grows, changes or becomes harder or is hardened. The epidermal corneum may grow and the corneum of the hair follicle may also grow more serious than the epidermal corneum to cause papules. The stratum (keratin) is formed by keratinization of the epidermal cells, and in a normal keratinization process, the nucleus cannot be found in the stratum corneum. In case of hyperkeratosis, the nucleus is well stained and thus is visible.

Prurigo is a skin disease accompanied by severe itching and a representative example thereof is Hebra prurigo. In addition, prurigo includes acute prurigo similar to eye blisters that occur frequently and rapidly decline in people of 15 to 30 years of age, tuberous prurigo looking like stubborn hives, pregnant prurigo that appears on the outside of the limb at 3 to 4 months of pregnancy and gradually becomes severe and the like. Pregnant prurigo occurs in parous women and is cured simultaneously with delivery.

Warts are caused by viral (HPV, human papillomavirus) infection resulting from a decrease in immunity. Warts have a small hemispherical shape with a size of about 2 to 5 mm and a smooth surface, are skin color or pink, include a depression in the center thereof, and often occur on the face, the back of the hand, the back of the foot, the body part, the anus, the perianal region, lips, mucosa, scalp and the like.

One embodiment of the present invention is characterized in that the extract inhibits abnormal differentiation of the (skin) stratum corneum.

In one embodiment of the present invention, the purple corn extract may be obtained by an extraction method selected from the group consisting of a solvent extraction method, an ultrasonic extraction method, a supercritical extraction method, a fermentation method and a traditional processing method, but the present invention is not limited thereto. The solvent extraction method may be carried out using a solvent selected from water, an organic solvent or a mixture thereof. Preferred is use of the solvent extraction method, but the present invention is not limited thereto. This method is advantageously capable of obtaining more active ingredients contained in the purple corn through the extraction process.

In one embodiment of the present invention, the organic solvent may be a C₁ to C₄ lower alcohol.

The composition of the present invention may further contain an adjuvant or additive. As such, the type and amount of the additive additionally contained in the pharmaceutical composition of the present invention are generally well-known in the related art.

In accordance with another embodiment of the present invention, there is provided a pharmaceutical preparation for preventing or treating a skin disease containing the pharmaceutical composition.

In one embodiment of the present invention, the pharmaceutical preparation may further contain at least one selected from the group consisting of a pharmaceutically acceptable carrier, a reinforcing agent and an excipient.

The composition containing a pharmaceutically acceptable carrier may be selected from various oral or parenteral formulations. The preparation may be carried out using a general diluent or excipient such as a filler, an extender, a binder, a wetting agent, a disintegrant or a surfactant. Solid preparations for oral administration include tablets, pills, powders, granules, capsules, and the like and the solid preparations may be prepared from a mixture of one or more excipients such as starch, calcium carbonate, sucrose, lactose and gelatin. In addition to simple excipients, a lubricant such as magnesium stearate and talc may also be used. Liquid preparations for oral administration include suspensions, solutions, emulsions, syrups and the like. The composition may contain a variety of excipients such as wetting agents, sweeteners, fragrances and preservatives, in addition to water and liquid paraffin, which are simple diluents commonly used. Preparations for parenteral administration include sterile aqueous solutions, non-aqueous solvents, suspensions, emulsions, freeze-dried preparations and suppositories. Examples of the non-aqueous solvents and suspending agents include propylene glycol, polyethylene glycol, vegetable oil such as olive oil, and injectable ester such as ethyl oleate. The base of the suppositories may be Witepsol, macrogol, tween 61, cacao butter, laurin butter, glycerogelatin and the like.

The composition of the present invention may be formulated into an appropriate application form, for example, a form for oral administration such as tablets, pills, powders, granules, capsules, suspensions, solutions, emulsions and syrups; and a form for parenteral administration such as sterile injectable aqueous solutions, non-aqueous solutions, suspensions, emulsions, freeze-dried preparations and suppositories.

The composition may be administered via any common route so long as it can reach the target tissue. The composition of the present invention may be administered intraperitoneally, intravenously, intraarterially, intramuscularly, intraosseously, intrauterinely, epidurally, subcutaneously, intradermally, orally, intranasally, intrapulmonarily or intrarectally, or injected intracerebroventricularly, but the present invention is not limited thereto. The composition may also be administered by any device capable of transferring the active ingredient to the target cell.

The composition of the present invention may be administered in a pharmaceutically effective amount.

As used herein, the term "pharmaceutically effective amount" means an amount sufficient to treat a disease at a reasonable benefit/risk ratio applicable to medical treatment, and the effective dosage level will vary depending on various factors including the type of a subject, severity, age, gender, the type of infected virus, the activity of the drug, sensitivity to the drug, the time of administration, the route of administration, the rate of excretion, the duration of the treatment, and co-administered drugs, and other factors well-known in the medical field. The composition of the present invention may be administered as an individual therapeutic agent or in combination with other therapeutic agents, and may be administered sequentially or simultaneously with conventional therapeutic agents. In addition, the composition may be administered in single or multiple doses. It is important to administer the drug in a minimal amount providing the maximum effect without side effects in consideration of all of the above factors and can be easily determined by those skilled in the art.

The composition of the present invention can be used alone or in combination with a method such as surgery, hormone therapy, drug therapy and use of biological response modifiers for the prevention or treatment of skin diseases.

The composition of the present invention can be used alone or in combination with a method such as surgery, hormone therapy, drug therapy and use of biological response modifiers for the prevention or treatment of skin diseases.

In one embodiment of the present invention, the purple corn extract according to the present invention may be present in the form of a powder or an extract in an amount of 0.001 to 90% by weight, preferably 0.001 to 10% by weight, and more preferably 0.1 to 1% by weight, with respect to 100% by weight of the total composition, based on solid contents. When the amount of the purple corn extract is less than 0.001% by weight with respect to the total composition, the effective ingredient of the purple corn extract is very little and thus the effect cannot be exhibited, and when the purple corn extract is present in more than 90% by weight, the effect of preventing or treating skin diseases may be deteriorated by a substance other than the present active ingredient.

The present invention relates to a method for preparing a composition for preventing or treating skin diseases including drying and pulverizing purple corn, extracting the pulverized material with a solvent selected from the group consisting of water, an organic solvent and a mixture thereof, and concentrating the extract under reduced pressure, followed by drying.

In one embodiment of the present invention, the organic solvent may be a C₁ to C₄ lower alcohol.

In one embodiment of the present invention, the drying may be drying selected from the group consisting of sun drying, hot air drying, evaporation drying, spray drying, freeze-drying, and a combination thereof.

In one embodiment of the present invention, the drying may be freeze-drying.

### [Advantageous effects]

One effect of the present invention is to provide a pharmaceutical composition for preventing or treating a skin disease, which is effective for the prevention or treatment of a skin disease without causing side effects by using a purple corn extract as a natural substance.

Another effect of the present invention is to provide a pharmaceutical composition for preventing or treating a skin disease, which is capable of inhibiting abnormal differentiation of the skin stratum corneum by containing a purple corn extract as an active ingredient.

### [Brief Description of the Drawings]

The above and other objects, features and other advantages of the present invention will be more clearly understood from the following detailed description taken in conjunction with the accompanying drawings, in which:
FIG. 1 is an image showing the process of 2-week application of a purple corn extract to an eczema patient ((A): before treatment and (B): after treatment);
FIG. 2 is an image showing a mitigation effect of skin lesion 8 days after applying a purple corn extract to an eczema experimental animal model;
FIG. 3 is a graph showing a mitigation effect of itching 2, 4, 6 and 8 days after applying the purple corn extract to the eczema experimental animal model;
FIG. 4 shows the results of measurement of IgE, an important biomarker of eczema, by extracting blood and collecting serum 8 days after applying the purple corn extract to the eczema experimental animal model;
FIGS. 5A to 5C show the effects of the purple corn extract on clinical changes of psoriasis such as erythema, keratin and thickness of skin lesions by applying the purple corn extract to an experimental animal model for treating psoriasis;
FIG. 6 is an image showing the effects of the purple corn extract on the mitigation effect of skin lesions by applying the purple corn extract to the experimental animal model for treating psoriasis;
FIGS. 7A and 7B show the effects of the purple corn extract on clinical changes of psoriasis such as erythema and thickness of ear skin lesions, by applying the purple corn extract to the experimental animal model for treating psoriasis;
FIGS. 8A and 8B are histological evaluation results of back (dorsal) skin lesions by applying the purple corn extract to the experimental animal model for treating psoriasis;
FIG. 9 shows the result of measurement of the ear weight after applying the purple corn extract to the experimental animal model for treating psoriasis, followed by dissection;
FIGS. 10A to 10D show the results of measurement of proportions of cells (FIG. 10A represents a proportion of helper T cells, FIG. 10B represents a proportion of cytotoxic T cells, FIG. 10C represents a proportion of regulatory T cells (CD4+CD25+) and FIG. 10D represents a proportion of CD11c cells) after applying the purple corn extract to the experimental animal model for treating psoriasis and then extracting the spleen;
FIGS. 11A to 11C show the effects of the purple corn extract on clinical changes of psoriasis such as erythema, keratin and thickness of skin lesions by applying the purple corn extract to an experimental animal model for preventing and treating psoriasis;
FIG. 12 is an image showing the effects of the purple corn extract on the mitigation of skin lesions by applying the purple corn extract to the experimental animal model for preventing and treating psoriasis;
FIGS. 13A and 13B show the effects of the purple corn extract on the clinical changes of psoriasis such as erythema and thickness of ear skin lesions by applying the purple corn extract to the experimental animal model for preventing and treating psoriasis;
FIGS. 14A and 14B are histological evaluation results of back skin lesions by applying the purple corn extract to the experimental animal model for preventing and treating psoriasis; and
FIGS. 15A to 15D are histological evaluation results of ear skin lesions by applying the purple corn extract to the experimental animal model for preventing and treating psoriasis.

### [Best mode]

The present invention provides a pharmaceutical composition for preventing or treating a skin disease containing a purple corn extract as an active ingredient.

### [Mode for Invention]

Hereinafter, the present invention will be described in more detail with reference to the following examples. However, the embodiments of the present invention may be embodied in a variety of different forms and should not be construed as limiting the scope of the present invention. In addition, the embodiments are suggested only to offer thorough and complete understanding of the present invention to those skilled in the art.

Preparation Example 1. Preparation of purple corn extract Purple corn [variety: *Zeamays. L*] was obtained from a corn test site (Gangwon Province, Korea). 5 kg of dried purple corns were pulverized, mixed with 30 L of a 30% ethanol-water solution in a glass chamber and then extracted at room temperature for 20 hours. The extract was collected and concentrated under a reduced pressure using a model EYELA N-1000 rotary evaporator (Tokyo Rikakikai, Tokyo, Japan) and lyophilized (yield: 410 g, 8.2%). The dried extract was stored at -20°C until used in the experiment below.

### Experimental Example 1. Evaluation of inhibition of abnormal differentiation of the skin stratum corneum

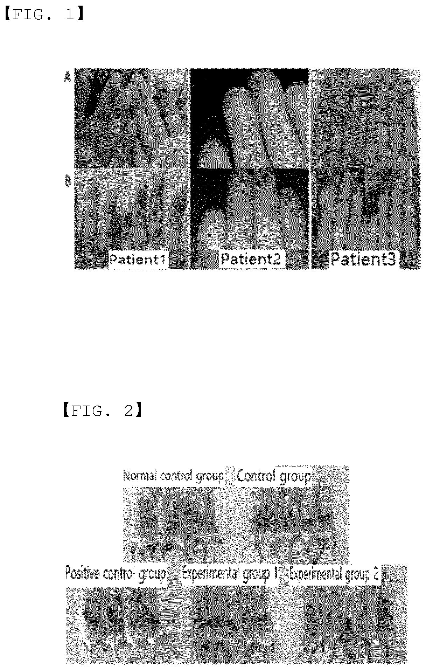

Three patients with eczema in their hands were recruited and the purple corn extract prepared in accordance with Preparation Example 1 was administered to the patients once a day for 2 weeks. Two weeks later, images of the hands of each patient were obtained to confirm changes in abnormal differentiation of the skin stratum corneum occurring in the hand (FIG. 1).

As shown in FIG. 1, all three patients showed alleviation of eczema, and cracks and red rashes were removed from patient 2 having shown the most severe eczema. Thus, it was demonstrated that the abnormal differentiation of the skin stratum corneum was suppressed by the purple corn extract.

### Experimental Example 2. Evaluation of mitigation of eczema (atopic) skin lesions using experimental animal model

First, animals (BALB/C mice) were anesthetized with pentobarbital and hair was removed therefrom. A solution of 1% 2,4-dinitrochlorobenzene (DNCB) in acetone (acetone:olive oil = 4:1) was applied to a gauze twice a day for 6 days, and a patch was attached to the skin of the experimental animal to induce eczema. After anesthesia, 100 µl of 1% 2,4-dinitrochlorobenzene was applied to the gauze twice a day to prepare a patch. The patch was attached to the skin to cause eczema (atopy). Then, as shown in Table 1 below, the composition was orally administered to the experimental animal model of each experimental group twice a day for 8 days. FIG. 2 shows the result of identifying the condition of the skin lesion associated with eczema.

**[Table 1]**

| Item | Orally administered composition |
|---|---|
| Normal control group | Saline |
| Control group | Saline |
| Positive control group | 3 mg/kg of dexamethasone |
| Experimental group 1 | 10 mg/kg of purple corn extract |
| Experimental group 2 | 30 mg/kg of purple corn extract |

As shown in FIG. 2, symptoms of eczema (atopy) occurring in the skin of experimental groups 1 and 2 orally administered the purple corn extract were alleviated as compared to a control group with no treatment. In addition, the symptoms of eczema were alleviated in experimental groups 1 and 2 like a positive control group orally administered the purple corn extract.

### Experimental Example 3. Evaluation of itching inhibition efficacy according to eczema (atopy) using experimental animal model

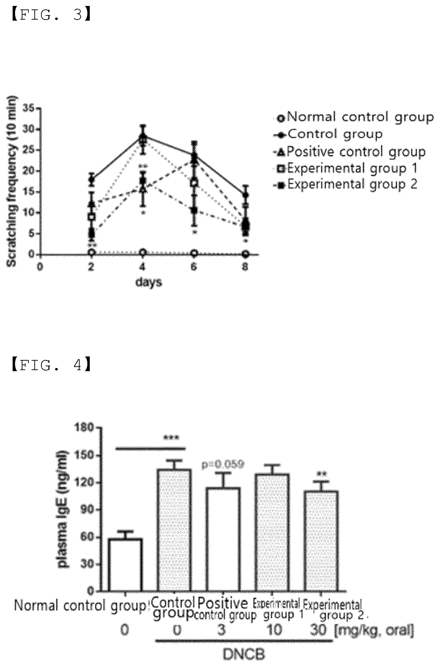

In order to evaluate the effect of suppressing itching caused by eczema, the number of times of scratches on 2, 4, 6, and 8 days was measured while orally administrating to the eczema-induced experimental animal model as shown in Table 1 above. Results are shown in FIG. 3.

As shown in FIG. 3, the control group had the highest number of scratches on the 4^{th} day. At this time, the number of scratches in the experimental group 2 was 50% less than that of the control group. On the other hand, the positive control group had the highest number of scratches on the 6^{th} day. At this time, the number of scratches was lower in the experimental groups 1 and 2 than in the positive control group. Thus, this indicated that the purple corn extract inhibited the itching symptoms.

### Experimental Example 4. Numerical measurement of biomarker IgE of eczema (atopy) using experimental animal model

After completion of Experimental Example 2, the skin tissue of the experimental animal model of each experimental group was sampled in two equal portions. One slice was fixed with 4% paraformaldehyde (at pH 7.4) and the other slice was frozen. Then, blood was sampled and the value of biomarker IgE thereof was measured. Results are shown in FIG. 4.

As shown in FIG. 4, experimental groups 1 and 2 were found to have values of IgE lowered to the same level as that of the positive control group administered dexamethasone, which is a currently commercially available drug.

### Experimental Example 5. Evaluation of effect of purple corn extract on treatment of psoriasis using experimental animal model for treating psoriasis

### 1. Preparation of experimental animal model for treating psoriasis

Psoriasis was induced by imiquimod cream (IMQ), a method reported by van der Fits *et al.* (2009). Seven week-old mice (BALB/C mice) as experimental animals were bred and domesticated in an experimental animal room for 7 days. The temperature of the feeding room was adjusted to 20.9 to 22.6°C, the relative humidity thereof was adjusted to 50 to 55%, the lighting period thereof was adjusted to 12 hours (08:00-20:00), and water and diet were fed freely. The back (dorsal) hair of the experimental animals was epilated using a depilatory cream (Veet, Oxy Reckitt Benckiser, Cedex, France). In consideration of the clinical symptoms of the back and ear, the experimental animal models were divided into four groups, as shown in Table 2 below. From the day after epilation, 62.5 mg of imiquimod cream was applied to the back and the right ear of the experimental groups excluding the normal control group for 6 days. At this time, 62.5 mg of Vaseline was applied to the back and the right ear of the normal control group every day.

**[Table 2]**

| Type | Application of imiquimod cream |
|---|---|
| Normal control group | X |
| Control group | ○ |
| Experimental group 1 | ○ |
| Experimental group 2 | ○ |

### 2. Clinical evaluation of skin lesions on purple corn extracts using experimental animal models for treating psoriasis

The composition was orally administered to four experimental groups of the experimental animal models for treating psoriasis for 7 days, as shown in Table 3 below. At this time, equivalent amounts of physiological saline were orally administered to the normal control group and the control group. The purple corn extract was dissolved in physiological saline and used.

**[Table 3]**

| Item | Orally administered composition |
|---|---|
| Normal control group | Physiological saline |
| Control group | Physiological saline |
| Experimental group 1 | 10 mg/kg of purple corn extract |
| Experimental group 2 | 30 mg/kg of purple corn extract |

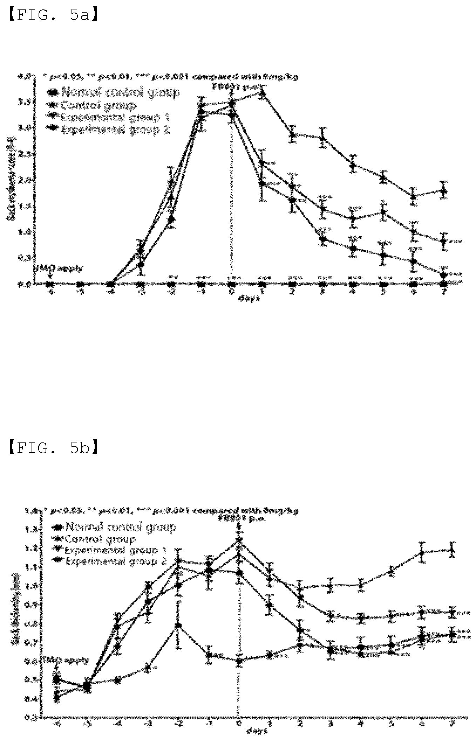

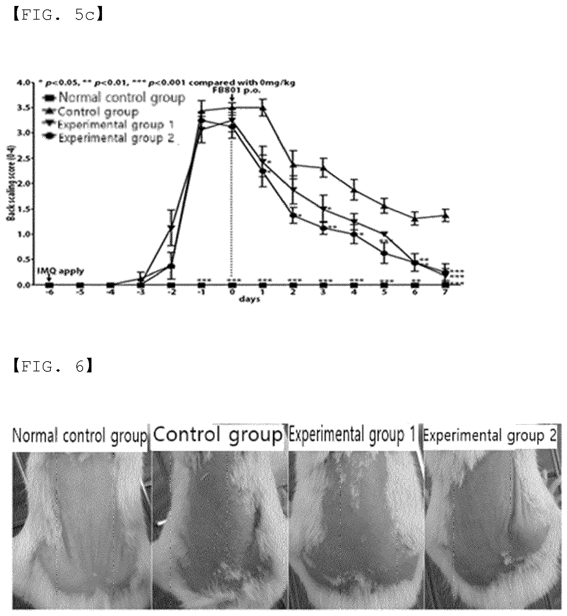

In addition, clinical changes of erythema, thickness and scaliness of skin lesions were observed for the four experimental groups of the experimental animal models for treating psoriasis for 7 days. The clinical skin severity score was assessed using a clinical visual evaluation method, commonly used for psoriasis evaluation. That is, erythema, thickness and scaliness were measured as visual evaluation items, and erythema and scaliness were each measured on three or more subjects having actual experience and were scored according to the following criteria: no symptoms (0 point), weak symptoms (1 point), moderate symptoms (2 points), severe symptoms (3 points), and very severe symptoms (4 points) and then were averaged. The thickness was measured using calipers. The results are shown in FIGS. 5 to 8.

As shown in FIG. 5, the degrees of erythema, thickness and scaliness of the back (dorsal) skin were the highest 6 days after application of the imiquimod cream. In all experimental animals, as the application of the imiquimod cream was stopped, the degrees of erythema, thickness and scaliness gradually decreased. In experimental groups 1 and 2, the degrees of erythema, thickness and scaliness were significantly lower than those of the control group. In addition, it was found that, as the concentration of orally administered purple corn extract increased, the degrees of erythema, thickness and scaliness decreased. It was found that, on the 7^{th} day after administration of the purple corn extract, symptoms of psoriasis in the experimental group 2 were alleviated to a similar level to that of the normal control group.

FIG. 6 is an image showing a mitigation effect of skin lesions through administration of a purple corn extract to a psoriasis-induced experimental animal model. Keratin, erythema and the like were observed in the back skin of the control group to which imiquimod cream was applied and only physiological saline was administered. On the other hand, the degrees of keratin and erythema on the back skin of experimental group 1 and experimental group 2 administered the purple corn extract were very good. Therefore, the purple corn extracts were found to be effective in the treatment of psoriasis.

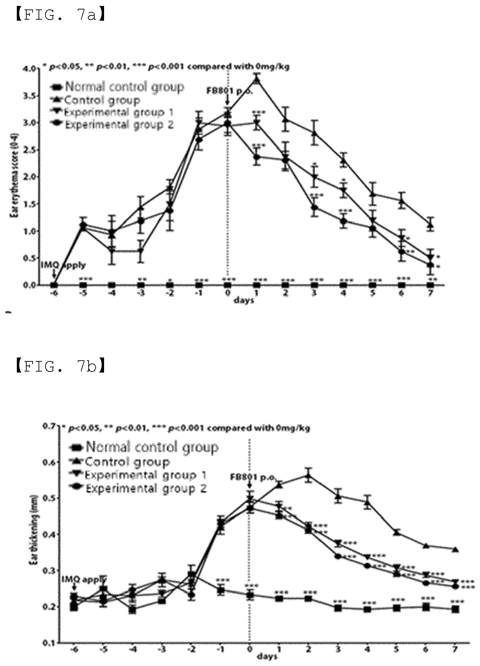

As shown in FIG. 7, the degrees of erythema and thickness of the right ear skin were similar to clinical evaluation results of the back skin. In other words, erythema and thickness were the highest 6 days after application of imiquimod cream, and erythema and thickness were increased in the control group 7 to 8 days after application. The experimental groups 1 and 2, immediately after oral administration of the purple corn extract, had significantly decreased erythema and thickness as compared to the control group.

### 3. Histological evaluation of skin lesions on purple corn extract using experimental animal model for treating psoriasis

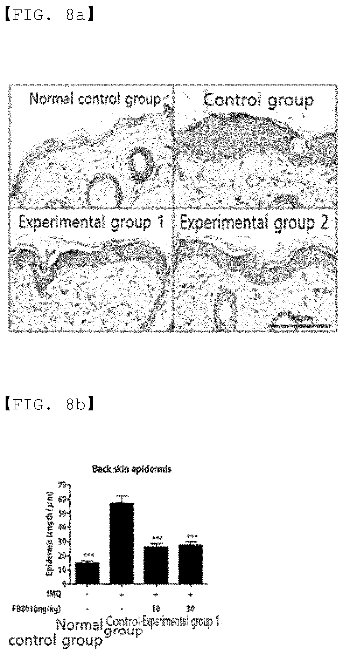

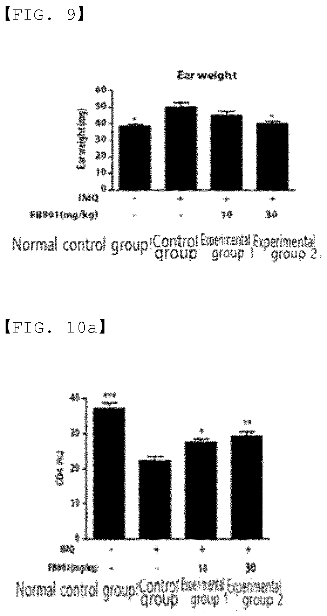

After dissection of the experimental animals of each experimental group, a part of the back skin was extracted and histologically observed by hematoxylin & eosin (H & E) staining. The results are shown in FIGS. 8 and 9.

As shown in FIG. 8, the thickness of the epidermis, which is one of the representative features of psoriasis, was significantly increased in the experimental animals of the control group as compared with the normal control group. On the other hand, in experimental group 1 and experimental group 2, the thickness of the epidermis was significantly decreased. In addition, the accumulation of inflammatory cells observed in the control group was found to be decreased on the back skin of the experimental animals to which the purple corn extract was administered.

FIG. 9 is a result of measurement of an ear weight to evaluate the degree of inflammation in the ear. The ear weight of each of experimental groups 1 and 2 was lower than that of the control group.

### 4. Evaluation of spleen and spleen lymphocyte changes by purple corn extract using experimental animal model for treating psoriasis

In the psoriasis model, it is known that the proportions of helper T cells and cytotoxic T cells of spleen cells decrease. Therefore, the effect of the purple corn extract on the changes of spleen and spleen lymphocytes was observed.

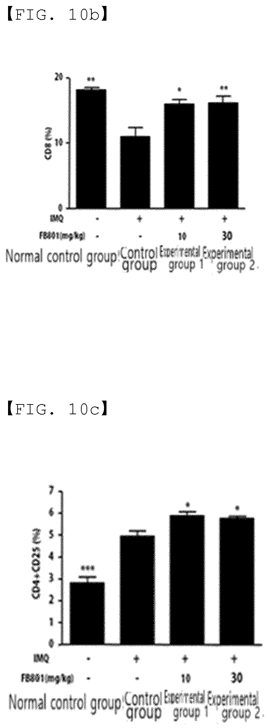

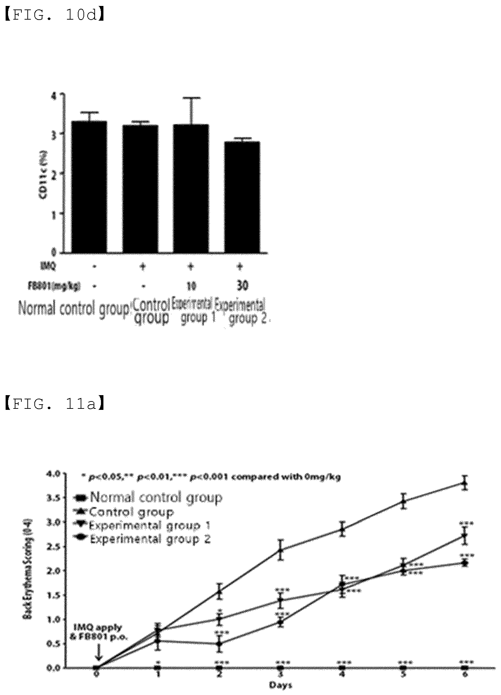

Specifically, the spleen was extracted from each experimental animal and was pulverized using a 40 µm stainless steel mesh (BD Falcon) in RPMI 1640 medium (Hyclone, Utah, USA) to obtain single cells. The RPMI 1640 medium was added to the pulverized single cell solution, followed by centrifuging at 4°C and 1,200 rpm for 5 minutes. Then, red blood cells were removed with RBC lysis buffer (eBIOSCIENCE) to obtain lymphocytes. The obtained lymphocytes were suspended in complete RPMI 1640 medium containing 10% fetal bovine serum (FBS), 100 units/mL penicillin and 100 µg/mL streptomycin, and then the number of cells was counted using a count & viability assay kit and a Muse cell analyzer. Based on the number of counted cells, a spleen cell suspension was prepared at a concentration of 5 × 10⁵ cells/tube, and a PE-anti CD4/FITC-anti CD3 monoclonal antibody, a PE-anti CD4/FITC-anti CD8α-monoclonal antibody, a PE-anti CD25/FITC-anti CD4 monoclonal antibody, and a CD11c-PE monoclonal antibody were added and reacted at 4°C for 30 minutes in the absence of light. Subsequently, the subpopulation proportion of spleen cells was measured using a fluorescence-activated cell sorting device (FACS). The results are shown in FIG. 10.

As can be seen from FIG. 10, the control group showed a significant decrease in the proportions of helper T cells and cytotoxic T cells as compared to the normal control group. It was observed that such a decrease was significantly restored by administration of the extract (FIGS. 10A and 10B). In addition, the proportion of regulatory T cells (CD4+CD25+), which generally have immunosuppressive activity, was significantly increased by administration of purple corn as compared to the control group (FIG. 10C). The proportion of CD11c cells in the spleen, known to increase due to psoriasis, showed a similar proportion in all the experimental groups (FIG. 10D). Thus, it was found that the purple corn extract affected a change in the proportion of T cells in the spleen.

### Experimental Example 6. Evaluation of effects of purple corn extract on treatment of psoriasis using experimental animal model for preventing and treating psoriasis

### 1. Preparation of experimental animal models for preventing and treating psoriasis

Psoriasis was induced by imiquimod cream, a method reported by van der Fits et al. (2009). Seven week-old mice (BALB/C mice) as experimental animals were bred and domesticated in an experimental animal room for 7 days. The temperature of the feeding room was adjusted to 20.9 to 22.6°C, the relative humidity thereof was adjusted to 50 to 55%, and the lighting period thereof was adjusted to 12 hours (08:00-20:00). The back hair of the experimental animals was epilated using a depilatory cream (Veet, Oxy Reckitt Benckiser, Cedex, France). In consideration of the clinical symptoms of the back and ear, the experimental animal models were divided into a normal control group, a control group, an experimental group 1 and an experimental group 2. From the day after epilation, 62.5 mg of imiquimod cream was applied to the back and the right ear of the experimental groups excluding the normal control group for 6 days. At this time, 62.5 mg of Vaseline was applied to the back and right ear of the normal control group every day. In addition, daily oral administration was carried out simultaneously with the application of the imiquimod cream, as shown in Table 4 below. At this time, equivalent amounts of physiological saline were orally administered to the normal control group and the control group. The purple corn extract was dissolved in physiological saline and used.

**[Table 4]**

| Type | Application of imiquimod cream | Orally administered composition |
|---|---|---|
| Normal control group | X | Physiological saline |
| Control group | ○ | Physiological saline |
| Experimental group 1 | ○ | 10 mg/kg of purple corn extract |
| Experimental group 2 | ○ | 30 mg/kg of purple corn extract |

### 2. Clinical evaluation of skin lesions on purple corn extracts using experimental animal models for preventing and treating psoriasis

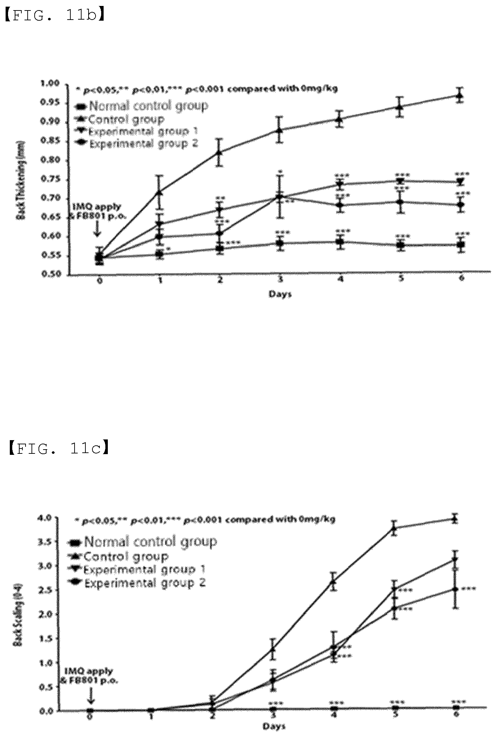

In addition, clinical changes of erythema, thickness and scaliness of skin lesions were observed for the four experimental groups of the experimental animal models for preventing and treating psoriasis for 7 days. The clinical skin severity score was assessed using a visual clinical evaluation method, commonly used for psoriasis evaluation. That is, erythema, thickness and scaliness were measured as visual evaluation items, and erythema and scaliness were each measured on three or more subjects having actual experience and were scored according to the following criteria: no symptoms (0 point), week symptoms (1 point), moderate symptoms (2 points), severe symptoms (3 points), and very severe symptoms (4 points) and then were averaged. The thickness was measured using calipers. The results are shown in FIGS. 11 to 13.

As shown in FIG. 11, the degrees of erythema (FIG. 11A), thickness (FIG. 11B) and scaliness (FIG. 11C) of the back skin in all experimental animals increased after application of the imiquimod cream. However, experimental groups 1 and 2 administered the purple corn extract had significantly decreased degrees of erythema, thickness and scaliness as compared to the control group.

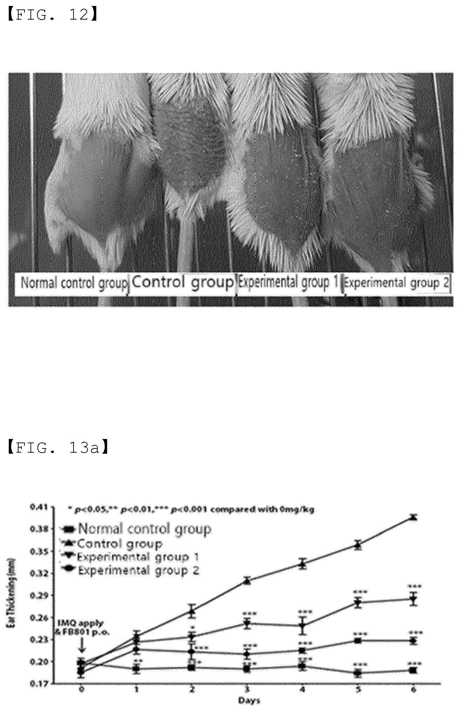

As shown in FIG. 12, severe keratin, erythema and the like were observed in the back skin of the control group to which the imiquimod cream was applied and only physiological saline was administered. On the other hand, the degrees of keratin and erythema on the back skin of experimental group 1 and experimental group 2 administered the purple corn extract were very good, as compared to the control group. Therefore, the purple corn extract was found to be effective in the prevention and treatment of psoriasis.

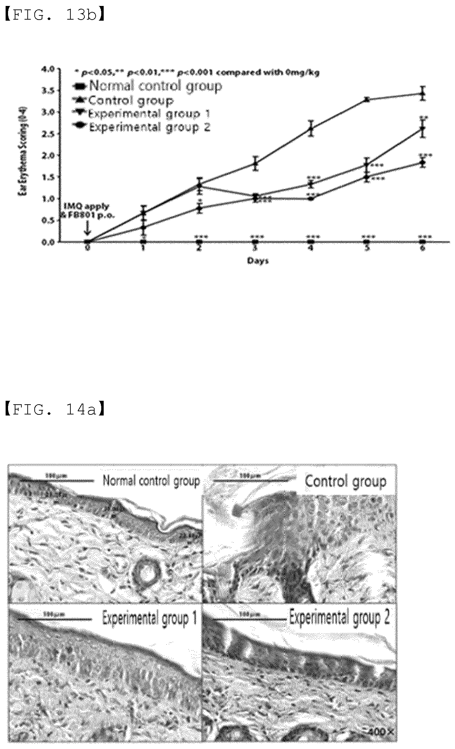

As shown in FIG. 13, the degrees of erythema and thickness of the right ear skin were similar to clinical evaluation results of the back skin. In the experimental groups 1 and 2, orally administered the purple corn extract, erythema and thickness are concentration-dependently decreased as compared to the control group.

### 3. Histological evaluation of skin lesions on purple corn extract using experimental animal model for treating psoriasis

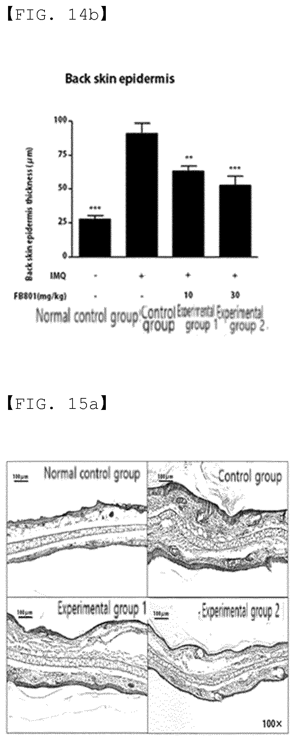

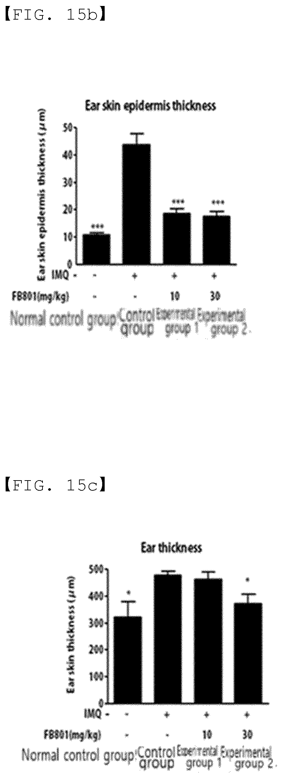

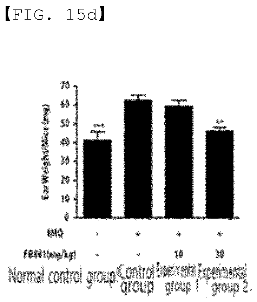

After dissection of the experimental animals of each experimental group, parts of the back and ear skin were extracted and histologically observed by hematoxylin & eosin (H & E) staining. The results are shown in FIGs. 14 and 15.

As shown in FIG. 14, the thickness of the epidermis on the back skin, which is one of the representative features of psoriasis, was significantly increased in the control group, to which the imiquimod cream was applied, as compared with the normal control group. On the other hand, in the experimental group 1 and the experimental group 2, to which the purple corn extract was orally administered, the thickness of the epidermis on the back skin was significantly decreased.

As can be seen from FIG. 15, the control group induced irritation due to the application of the imiquimod cream and thus had an increase in the thickness of the ear skin and the thickness of the epidermis. In addition, many inflammatory cells were precipitated in the control group. On the other hand, experimental groups 1 and 2, to which the purple corn extract was orally administered, alleviated the degree of inflammatory reactions and thus showed a decrease in the thickness of the ear skin and the thickness of the epidermis. In addition, the experimental groups 1 and 2 showed significantly low precipitation levels of inflammatory cells, as compared to the control group. In particular, the level of inflammatory cells in the experimental group 2 was much lower than that of the control group.

In addition, FIG. 15 is a result of measurement of an ear weight to evaluate the degree of inflammation in the ear. The ear weight of each of experimental groups 1 and 2 was lower than that of the control group.

Although the preferred embodiments of the present invention have been disclosed in detail for illustrative purposes, those skilled in the art will appreciate that various modifications, additions and substitutions are possible, without departing from the scope and spirit of the invention as disclosed in the accompanying claims.

### [Industrial Applicability]

The present invention relates to a pharmaceutical composition for preventing or treating a skin disease containing a purple corn extract, and more particularly, to a pharmaceutical composition for preventing or treating a skin disease containing a purple corn extract that has a potent effect of preventing or treating at least one skin disease selected from the group consisting of atopy, psoriasis, eczema, keratosis, prurigo and warts, using the purple corn extract.

## Claims

1. A pharmaceutical composition for preventing or treating a skin disease comprising a purple corn extract as an active ingredient.

2. The pharmaceutical composition according to claim 1, wherein the skin disease comprises at least one selected from the group consisting of atopy, psoriasis, eczema, keratosis, prurigo and warts.

3. The pharmaceutical composition according to claim 2, wherein the skin disease is psoriasis.

4. The pharmaceutical composition according to claim 2, wherein the skin disease is eczema.

5. The pharmaceutical composition according to claim 2, wherein the skin disease is atopy.

6. The pharmaceutical composition according to claim 2, wherein the purple corn extract is present in an amount of 0.001 to 90% by weight, with respect to a weight of the total composition.

7. The pharmaceutical composition according to claim 1, wherein the purple corn extract suppresses abnormal differentiation of a skin stratum corneum.

8. The pharmaceutical composition according to claim 1, wherein the purple corn extract is obtained by an extraction method selected from the group consisting of a solvent extraction method, an ultrasonic extraction method, a supercritical extraction method, a fermentation method and a traditional processing method.

9. The pharmaceutical composition according to claim 8, wherein the solvent extraction method is carried out using a solvent selected from the group consisting of water, an organic solvent and a mixture thereof.

10. The pharmaceutical composition according to claim 9, wherein the organic solvent is a C₁ to C₄ lower alcohol.

11. The pharmaceutical composition according to claim 1, further comprising an adjuvant or additive.

12. A pharmaceutical preparation for preventing or treating a skin disease comprising the pharmaceutical composition according to any one of claims 1 to 11.

13. The pharmaceutical preparation according to claim 12, wherein the pharmaceutical preparation is selected from the group consisting of a tablet, a pill, a powder, a capsule, a syrup and an emulsion.

14. The pharmaceutical preparation according to claim 12, further comprising at least one selected from the group consisting of a pharmaceutically acceptable carrier, a reinforcing agent and an excipient.

15. A method for preparing the pharmaceutical composition for preventing or treating a skin disease according to claim 1 comprising:
drying and pulverizing purple corn;
extracting the pulverized material with a solvent selected from the group consisting of water, an organic solvent and a mixture thereof; and
concentrating the extract under reduced pressure, followed by drying.

16. The method according to claim 15, wherein the organic solvent is a C₁ to C₄ lower alcohol.

17. The method according to claim 15, wherein the drying is selected from the group consisting of sun drying, hot air drying, evaporation drying, spray drying, freeze-drying, and a combination thereof.

18. The method according to claim 17, wherein the drying is freeze-drying.
